# EUROPEAN PATENT SPECIFICATION

(11) **EP 3 100 046 B1**
(45) Date of publication and mention of the grant of the patent: **20.04.2022**
(21) Application number: 15740999.6
(22) Date of filing: 26.01.2015
(51) Int. Cl.: G01N 33/574

(54) **DETECTION OF PROSTATE SPECIFIC MEMBRANE ANTIGEN (PSMA) EXPRESSION ON CIRCULATING TUMOR CELLS (CTC)**
ERKENNUNG VON PROSTATA-SPEZIFISCHER MEMBRANANTIGENEXPRESSION IN ZIRKULIERENDEN TUMORZELLEN
DÉTECTION DE L'EXPRESSION DE L'ANTIGÈNE MEMBRANAIRE SPÉCIFIQUE DE LA PROSTATE (PSMA) SUR DES CELLULES TUMORALES CIRCULANTES (CTC)

(30) Priority: 27.01.2014 US 201461932117 P; 30.01.2014 US 201461933771 P
(43) Date of publication of application: 07.12.2016
(73) Proprietor: Epic Sciences, Inc., San Diego, CA 92121 (US)
(72) Inventor: DITTAMORE, Ryan, San Diego, CA 92121 (US)
(74) Representative: Jones Day
(86) International application number: PCT/US2015/012957
(87) International publication number: WO 2015/112999

(56) References cited:
- WO-A1-2011/093927
- WO-A1-2013/181532
- WO-A1-2013/181532
- WO-A1-2014/008155
- WO-A2-2012/103025
- US-A1- 2002 098 535
- US-A1- 2012 276 555
- US-A1- 2012 276 555
- LARSON CHRISTOPHER J ET AL: "Apoptosis of circulating tumor cells in prostate cancer patients", CYTOMETRY, ALAN LISS, NEW YORK, US, vol. 62A, no. 1, 1 November 2004 (2004-11-01), pages 46-53, XP002451688, ISSN: 0196-4763
- Anonymous: "Circulating tumor cell", Wikipedia, 13 January 2015 (2015-01-13), XP055472147, Retrieved from the Internet: URL:https://en.wikipedia.org/w/index.php?t itle=Circulating_tumor_cell&oldid=64223529 5 [retrieved on 2018-05-03]
- RACILA E ET AL: "Detection and charcterization of carcinoma cells in the blood", PROCEEDINGS OF THE NATIONAL ACADEMY OF SCIENCES, NATIONAL ACADEMY OF SCIENCES, US, vol. 95, 1 April 1998 (1998-04-01), pages 4589-4594, XP002132943, ISSN: 0027-8424, DOI: 10.1073/PNAS.95.8.4589
- PETER SIDAWAY: "Non-traditional CTCs indicate prognosis : Urological cancer", NATURE REVIEWS CLINICAL ONCOLOGY, vol. 13, no. 10, 7 September 2016 (2016-09-07), pages 592-592, XP055472116, NY, US ISSN: 1759-4774, DOI: 10.1038/nrclinonc.2016.146
- Karen A Autio ET AL: "Heterogeneity of prostate-specific membrane antigen (PSMA) expression in classic and apoptotic circulating tumor cells (CTC) in metastatic castration-resistant prostate cancer (mCRPC).", Journal of Clinical Oncology: Vol 32, No 4_suppl, 1 February 2014 (2014-02-01), XP055381023, DOI: 10.1200/jco.2014.32.4_suppl.198 Retrieved from the Internet: URL:http://ascopubs.org/doi/abs/10.1200/jc o.2014.32.4_suppl.198 [retrieved on 2017-06-13]
- T. A. Yap ET AL: "Circulating Tumor Cells: A Multifunctional Biomarker", CLINICAL CANCER RESEARCH, vol. 20, no. 10, 14 May 2014 (2014-05-14), pages 2553-2568, XP055568616, US ISSN: 1078-0432, DOI: 10.1158/1078-0432.CCR-13-2664
- E. ROSSI ET AL: "M30 Neoepitope Expression in Epithelial Cancer: Quantification of Apoptosis in Circulating Tumor Cells by CellSearch Analysis", CLINICAL CANCER RESEARCH, vol. 16, no. 21, 26 October 2010 (2010-10-26), pages 5233-5243, XP055385379, US ISSN: 1078-0432, DOI: 10.1158/1078-0432.CCR-10-1449

## Description

The present disclosure relates generally to accurate and non-invasive methods for detecting prostate specific membrane antigen (PSMA) on CTCs.

### BACKGROUND

Prostate cancer is the most commonly diagnosed solid organ malignancy in the United States (US) and remains the second leading cause of cancer deaths among American men. In 2014 alone, the projected incidence of prostate cancer is 233,000 cases with deaths occurring in 29,480 men, making metastatic prostate cancer therapy truly an unmet medical need. Siegel et al., 2014. CA Cancer J Clin. 2014;64(1):9-29. Epidemiological studies from Europe show comparable data with an estimated incidence of 416700 new cases in 2012, representing 22.8% of cancer diagnoses in men. In total, 92200 prostate cancer deaths are expected, making it one of the three cancers men are most likely to die from, with a mortality rate of 9.5%

Despite the proven success of hormonal therapy for prostate cancer using chemical or surgical castration, most patients eventually will progress to a phase of the disease that is metastatic and shows resistance to further hormonal manipulation. This has been termed metastatic castration-resistant prostate cancer (mCRPC). Despite this designation, however, there is evidence that androgen receptor (AR)-mediated signaling and gene expression can persist in mCRPC, even in the face of castrate levels of androgen. This may be due in part to the upregulation of enzymes involved in androgen synthesis, the overexpression of AR, or the emergence of mutant ARs with promiscuous recognition of various steroidal ligands. Treatment of patients with mCRPC remains a significant clinical challenge.

Prior to 2004, there was no treatment proven to improve survival for men with mCRPC. The treatment of patients with mitoxantrone with prednisone or hydrocortisone was aimed only at alleviating pain and improving quality of life, but there was no benefit in terms of overall survival (OS). In 2004, the results of two major phase 3 clinical trials, TAX 327 and SWOG (Southwest Oncology Group) 9916, established Taxotere^{®} (docetaxel) as a primary chemotherapeutic option for patients with mCRPC. Additional hormonal treatment with androgen receptor (AR) targeted therapies, chemotherapy, combination therapies, and immunotherapy, has been investigated for mCRPC, and recent results have offered additional options in this difficult-to-treat patient group. With the advent of exponential growth of novel agents tested and approved for the treatment of patients with metastatic castration-resistant prostate cancer (mCRPC) in the last 5 years alone, issues regarding the optimal sequencing or combination of these agents have arisen. Several guidelines exist that help direct clinicians as to the best sequencing approach and most would evaluate presence or lack of symptoms, performance status, as well as burden of disease to help determine the best sequencing for these agents. Mohler et al., J Natl Compr Canc Netw. 2013;11(12):1471-1479; Mohler et al., J Natl Compr Cane Netw. 2014; 12(5): 686-718; Cookson et al. , J Urol. 2013;190(2):429-438. Currently, approved treatments consist of taxane-class cytotoxic agents such as Taxotere^{®} (docetaxel) and Jevtana^{®} (cabazitaxel), and anti-androgen hormonal therapy drugs such as Zytiga^{®} (arbiterone, blocks androgen production) or Xtandi^{®} (enzalutamide, an androgen receptor (AR) inhibitor).

The challenge for clinicians is to decide the best sequence for administering these therapies to provide the greatest benefit to patients. However, therapy failure remains a significant challenge based on heterogeneous responses to therapies across patients and in light of cross-resistance from each agent. Mezynski et al. , Ann Oncol. 2012;23(11):2943-2947;. Noonan et al., Ann Oncol. 2013;24(7):1802-1807; Pezaro et al., Eur Urol. 2014, 66( 3): 459-465. In addition, patients may lose the therapeutic window to gain substantial benefit from each drug that has been proven to provide overall survival gains. Hence, better methods of identifying the target populations who have the most potential to benefit from targeted therapies remain an important goal.

PSMA is a dimeric type II integral membrane glycoprotein, highly expressed on prostate cancer cells. It has been shown to be a predictor for prostate cancer progression and for prognosis of prostate cancer. For example, elevated PSMA levels are seen in aggressive forms of the disease, i.e., metastatic and higher-grade prostate cancers. High-level PSMA expression is correlated with early PSA recurrence in surgically treated prostate cancer. PSMA correlates with the aggressiveness of the disease, androgen receptor (AR) targeted therapy failure and progressive disease, and thereby strongly support PSMA as a target for prostate cancer characterization and subsequent therapy. The ability to detect PSMA levels on CTCs comprising high definition imaging of plated nucleated cells can identify patients likely to benefit from therapeutic approaches that target PSMA on the cell surface of CTCs. Hence, it may be feasible to deliver PSMA targeted agents to the tumor and its microvasculature to (1) selectively destroy the vessels perfusing the tumor tissue, (2) achieve high regional doses of drugs to overcome tumor resistance, and (3) spare normal tissues, which typically lack PSMA expression. The ability to detect PSMA levels on CTCs comprising high definition imaging of plated nucleated cells also can identify patients likely have increased risk of AR targeted therapy failure.

Circulating tumor cells (CTCs) represent a significant advance in cancer diagnosis made even more attractive by their non-invasive measurement. Cristofanilli et al., N Engl J Med 2004, 351:781-91. CTCs released from either a primary tumor or its metastatic sites hold important information about the biology of the tumor. Historically, the extremely low levels of CTCs in the bloodstream combined with their unknown phenotype has significantly impeded their detection and limited their clinical utility. A variety of technologies have recently emerged for detection, isolation and characterization of CTCs in order to utilize their information. CTCs have the potential to provide a non-invasive means of assessing progressive cancers in real time during therapy, and further, to help direct therapy by monitoring phenotypic physiological and genetic changes that occur in response to therapy. In most advanced prostate cancer patients, the primary tumor has been removed, and CTCs are expected to consist of cells shed from metastases, providing a "liquid biopsy." While CTCs are traditionally defined as EpCAM/cytokeratin positive (CK+) cells, CD45-, and morphologically distinct, recent evidence suggests that other populations of CTC candidates exist including cells that are EpCAM/cytokeratin negative (CK-) or cells smaller in size than traditional CTCs. These findings regarding the heterogeneity of the CTC population, suggest that enrichment-free CTC platforms are favorable over positive selection techniques that isolate CTCs based on size, density, or EpCAM positivity that are prone to miss important

CTC subpopulations. US 2002/098535 A1 relates to the characterization of isolated cancer cells from a body fluid sample into classes and subclasses comprising terminal calls, proliferative cells and intermediate cells.

A need exists to develop accurate and non-invasive methods for detecting the emergence and monitoring PSMA expression on diverse types of CTCs in patients with mCRPC.

The present invention addresses this need assay by providing an assay to detect and quantify PSMA expression on diverse types of CTCs in patients with mCRPC. Related advantages are provided as well.

### SUMMARY

The present invention provides a method for detecting prostate specific membrane antigen (PSMA) on circulating tumor cells (CTCs) obtained from a patient afflicted with prostate cancer as defined in the appended claims and comprising (a) performing a direct analysis comprising immunofluorescent staining and morphological characterization of nucleated cells in a blood sample obtained from the patient to detect circulating tumor cells (CTC), and (b) determining the number of CTCs expressing PSMA.

According to the method, the direct analysis in step (a) identifies more than one CTC subpopulation expressing PSMA : a first CTC subpopulation expressing PSMA comprises CK+, CD45-, traditional CTCs, and a second CTC subpopulation expressing PSMA comprises CK+, CD45-, apoptotic CTCs. In some embodiments, the prostate cancer is metastatic castration-resistant prostate cancer (mCRPC). In additional embodiments, the immunofluorescent staining of nucleated cells comprises pan cytokeratin (CK), cluster of differentiation (CD) 45, and diamidino-2-phenylindole (DAPI).

In further embodiments, determining the presence of a CTC subpopulation expressing PSMA in step (b) comp rises analysis of the CTCs detected in step (a) at the single cell level. The method for detecting prostate specific membrane antigen (PSMA) on circulating tumor cells (CTCs) may comprise molecular characterization of the CTCs. The method for detecting prostate specific membrane antigen (PSMA) on circulating tumor cells (CTCs) may comprise analysis of the CTC subpopulation expressing PSMA to identify clonal subtypes within the subpopulation.

In additional embodiments, the method for detecting prostate specific membrane antigen (PSMA) on circulating tumor cells (CTCs) obtained from a patient afflicted with prostate cancer comprises a step comparing the number of CTCs expressing PSMA to a reference value. In further embodiments, the method for detecting PSMA on circulating tumor cells (CTCs) obtained from a patient afflicted with prostate cancer comprises determining the proportion of CTCs that comprise CTCs expressing PSMA. In some embodiments, the proportion of CTCs that comprise CTCs expressing PSMA is compared to a reference value. In further embodiments, a proportion of CTCs expressing PSMA above the reference value identifies the patient as a candidate for PSMA targeted therapy. In additional embodiments, a proportion of CTCs expressing PSMA above the reference value is indicative of resistance to androgen receptor (AR) targeted therapy.

The method of the invetion may be used for identifying a patient afflicted with prostate cancer as a candidate for PSMA targeted therapy.

The method of the invention may also be used for predicting resistance to androgen receptor (AR) targeted therapy a patient afflicted with prostate cancer.

### BRIEF DESCRIPTION OF THE DRAWINGS

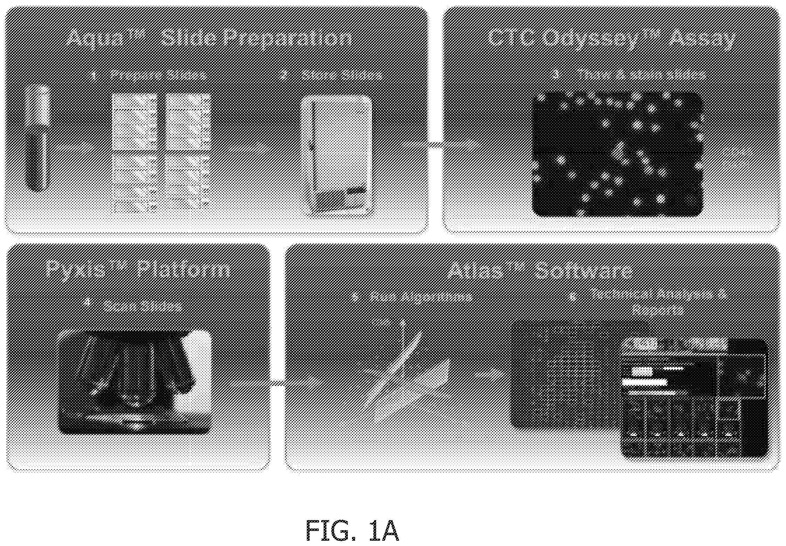

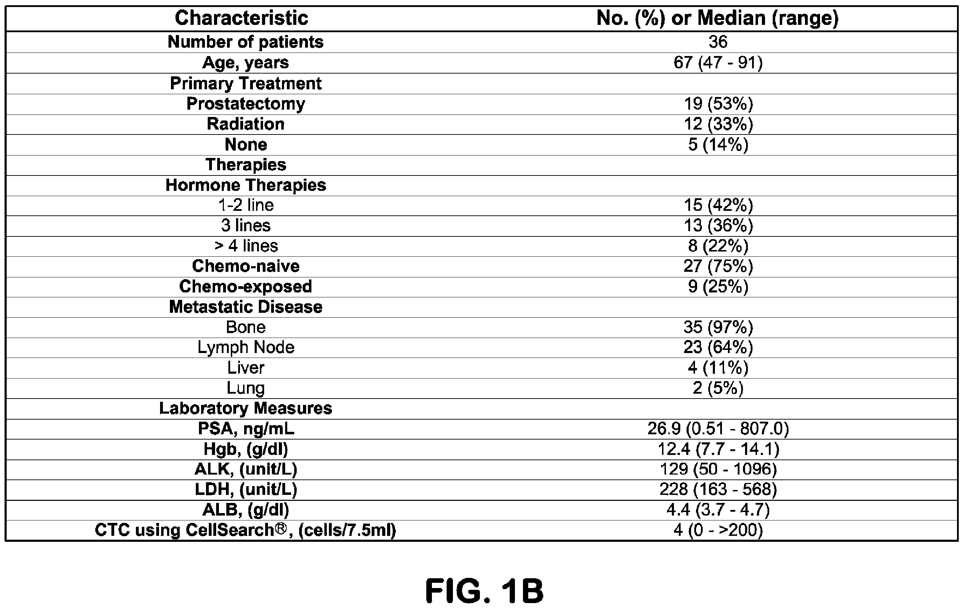

Figures 1A and 1B, show methods relating to performing the embodiments described herein and describes the exemplified study population. Figure 1A shows a schematic of a representative CTC collection and detection process: (1) nucleated cells from blood sample placed onto slides; (2) slides stored in -80°C biorepository; (3) slides stained with CK, CD45, DAPI and AR; (4) slides scanned; (5) multi-parametric digital pathology algorithms run; (6) software and human reader confirmation of CTCs and quantitation of biomarker expression. Figure 1B shows information on the study population. Demographic and clinical characteristics, including current and prior therapy history, PSA, and concomitant enumeration by CellSearch^{®}, of patients at the time of inclusion in the study are shown in the left and right panels. Thirty six patients (pts) with metastatic castration resistant prostate cancer (mCRPC) were included in the study.

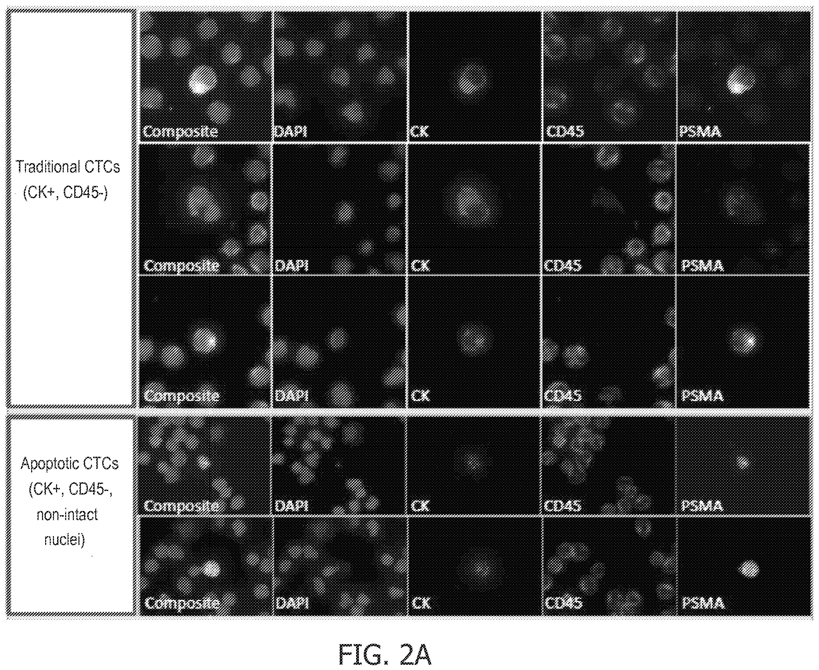

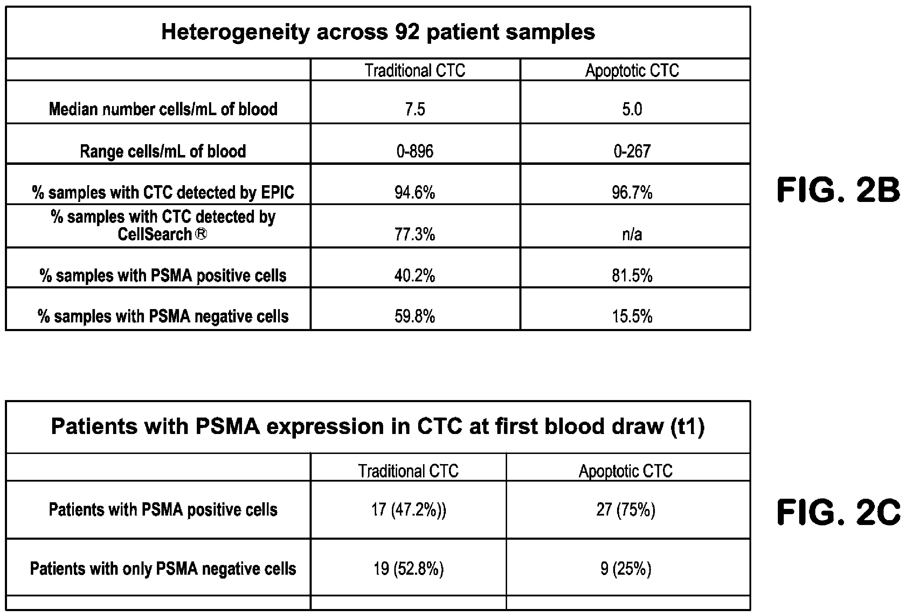

Figures 2A, 2B and 2C show PSMA heterogeneity in CTCs. Figure 2A shows immunofluorescence images of heterogeneity of PSMA expression in traditional CTCs and apoptotic CTCs from a single blood draw. Figure 2B shows a table that describes heterogeneity across ninety two patient samples as detected by Epic versus CellSearch^{®}. Figure 2C shows a table that details percentages of patients with PSMA expression on CTCs at the first blood draw.

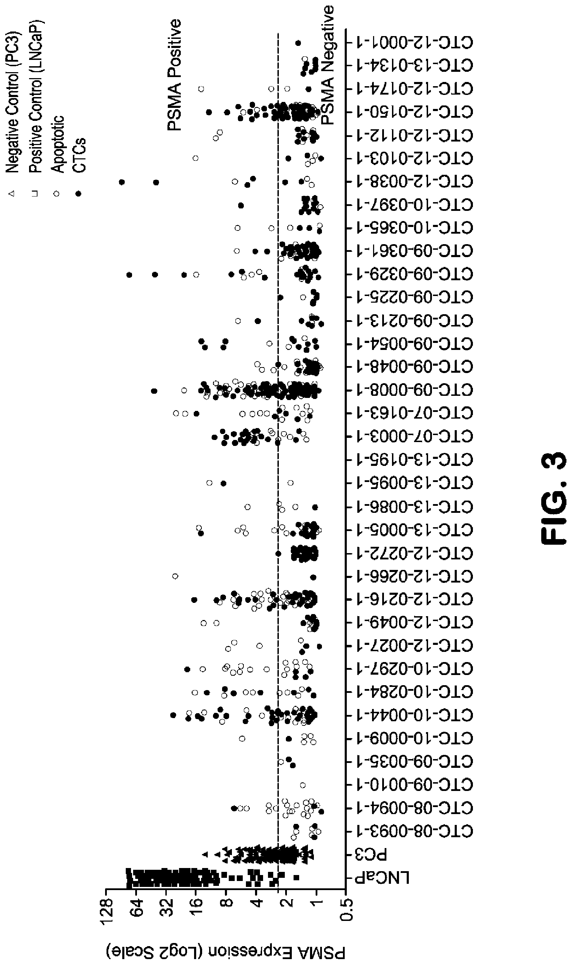

Figure 3 shows PSMA expression per CTC and apoptotic CTC as calculated by Epic software plotted for each patient.

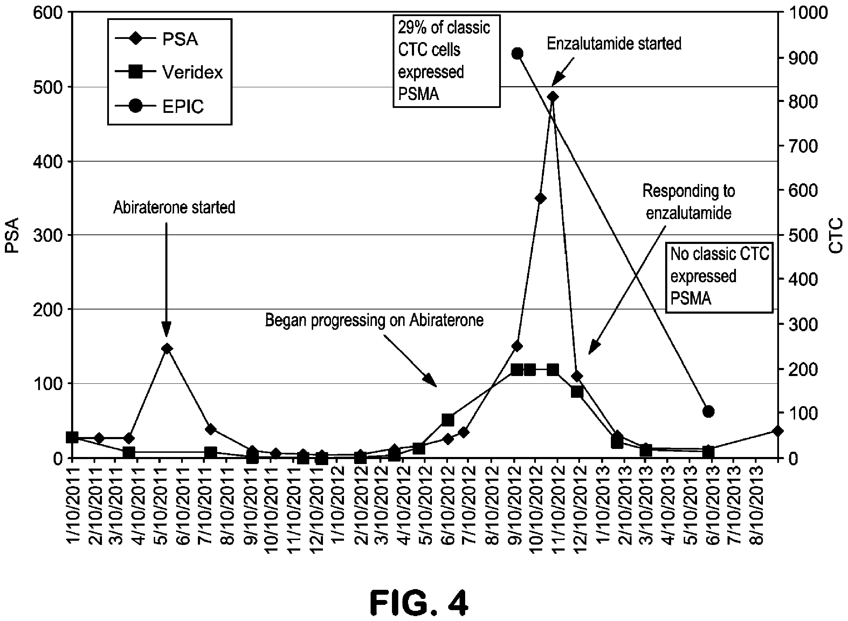

Figure 4 shows a CTC profile in a patient progressing on abiraterone and subsequent response to enzalutamide, with corresponding conversion from PSMA+ CTCs to PSMA- CTCs and decline in absolute CTCs.

### DETAILED DESCRIPTION

The present disclosure is based, in part, on the unexpected discovery that detection of PSMA on CTCs surface comprising high definition imaging of plated nucleated cells based on a direct analysis comprising immunofluorescent staining and morphological characteristics of the nucleated cells can be used to determine PSMA heterogeneity and dynamic changes in PSMA expression over time.

The present invention provides a method for detecting PSMA on circulating tumor cells (CTCs) obtained from a patient afflicted with prostate cancer comprising (a) performing a direct analysis comprising immunofluorescent staining and morphological characterization of nucleated cell in a blood sample obtained from the patient to detect circulating tumor cells (CTC), and (b) determining the number of CTCs expressing PSMA as defined in the appended claims.

In some embodiments of the methods disclosed herein, the immunofluorescent (CD45) staining of nucleated cells comprises pan cytokeratin (CK), cluster of differentiation (CD) 45, and diamidino-2-phenylindole (DAPI). According to the method of the invention, the direct analysis in step (a) identifies more than one CTC subpopulation expressing PSMA. ^{A} first CTC subpopulation expressing PSMA comprises CK+, CD45-, traditional CTCs, and a second CTC subpopulation expressing PSMA comprises CK+, CD45-, apoptotic CTCs. In some embodiments of the methods disclosed herein, the apoptotic CTCs lack intact nuclei.

In some embodiments of the methods disclosed herein, the presence of a CTC subpopulation expressing PSMA in step (b) comprises analysis of the CTCs detected in step (a) at the single cell level. The methods disclosed herein may further comprise molecular characterization of the CTCs. The methods disclosed herein may further comprise molecular analysis of the CTC subpopulation expressing PSMA to identify clonal subtypes within said subpopulation. The identification of previously unknown clonal subtypes expressing PSMA can further enable new methods for identifying patients that are prone to be resistant or in the process of acquiring resistance to AR targeted therapy.

In some aspects of the invention the prostate cancer is castration-resistant prostate cancer (CRPC). In further aspects of the invention the prostate cancer is metastatic castration-resistant prostate cancer (mCRPC).

As described above, the present invention provides a method for detecting PSMA on circulating tumor cells (CTCs) obtained from a patient afflicted with prostate cancer comprising (a) performing a direct analysis comprising immunofluorescent staining and morphological characterization of nucleated cells in a blood sample obtained from the patient to detect circulating tumor cells (CTC), and (b) determining the number of CTCs expressing PSMA. According to the method of the invention, the direct analysis in step (a) identifies more than one CTC subpopulation expressing PSMA: a first CTC subpopulation expressing PSMA comprises CK+, CD45-, traditional CTCs, and a second CTC subpopulation expressing PSMA comprises CK+, CD45-, apoptotic CTCs. In some embodiments, the methods disclosed herein further comprise a step comparing the number of CTCs expressing PSMA to a reference value. In some embodiments, the methods disclosed herein further comprise determining the proportion of CTCs that comprise CTCs expressing PSMA. In some embodiments of the claimed methods, the proportion of CTCs that comprise CTCs expressing PSMA is compared to a reference value. In related embodiments, the proportion of CTCs expressing PSMA above said reference value identifies the patient as a candidate for PSMA targeted therapy. In further embodiments, the proportion of CTCs expressing PSMA above said reference value is indicative of resistance to androgen receptor (AR) targeted therapy.

In some aspects of the invention, the methods of the invention comprises determining the proportion of CTCs that comprise the first subpopulation of CTCs expressing PSMA. In some aspects of the invention, the methods of the invention comprises determining the proportion of CTCs that comprise the second subpopulation of CTCs expressing PSMA. In some aspects of the invention, a proportion of CTCs that comprise CTCs expressing PSMA is compared to a reference value. In some aspects of the invention, a proportion of CTCs expressing PSMA above said reference value identifies the patient as a candidate for PSMA targeted therapy. In some aspects of the invention, a proportion of CTCs expressing PSMA above said reference value is indicative of resistance to androgen receptor (AR) targeted therapy.

The method of the invention may be used for identifying a patient afflicted with prostate cancer as a candidate for PSMA targeted therapy.

The method of the invention may also be used for predicting resistance to androgen receptor (AR) targeted therapy a patient afflicted with prostate cancer.

It must be noted that, as used in this specification and the appended claims, the singular forms "a", "an" and "the" include plural referents unless the content clearly dictates otherwise. Thus, for example, reference to "a CTC" includes a mixture of two or more CTCs, and the like.

The term "about," particularly in reference to a given quantity, is meant to encompass deviations of plus or minus five percent.

As used in this application, including the appended claims, the singular forms "a," "an," and "the" include plural references, unless the content clearly dictates otherwise, and are used interchangeably with "at least one" and "one or more."

As used herein, the terms "comprises," "comprising," "includes," "including," "contains," "containing," and any variations thereof, are intended to cover a non-exclusive inclusion, such that a process, method, product-by-process, or composition of matter that comprises, includes, or contains an element or list of elements does not include only those elements but can include other elements not expressly listed or inherent to such process, method, product-by-process, or composition of matter.

The term "patient," as used herein preferably refers to a human, but also encompasses other mammals. It is noted that, as used herein, the terms "organism," "individual," "subject," or "patient" are used as synonyms and interchangeably.

As used herein, the term "circulating tumor cell" or "CTC" is meant to encompass any rare cell that is present in a biological sample and that is related to prostate cancer. CTCs, which can be present as single cells or in clusters of CTCs, are often epithelial cells shed from solid tumors found in very low concentrations in the circulation of patients. CTCs include "traditional CTCs," which are cytokeratin positive (CK+), CD45 negative (CD45-), contain a DAPI nucleus, and are morphologically distinct from surrounding white blood cells. The term also encompasses "non-traditional CTCs" which differ from a traditional CTC in at least one characteristic. Non-traditional CTCs include the five CTC subpopulations, including CTC clusters, CK negative (CK⁻) CTCs that are positive at least one additional biomarker that allows classification as a CTC , small CTCs, nucleoli ⁺ CTCs and CK speckled CTCs. As used herein, the term "CTC cluster" means two or more CTCs with touching cell membranes. As used herein, a "subpopulation" refers to two or more CTCs that share one or more morphological and/or immunofluorescent characteristics. As disclosed herein, CTC subpopulations associated with CRPC comprise comprises CK+, CD45-, traditional CTCs and CK+, CD45-, apoptotic CTCs.

The majority of patients with systemic prostate cancer treated with androgen deprivation therapy (ADT), also referred to a "primary" hormone therapy in the context of prostate cancer, will develop castration-resistant prostate cancer (CRPC). Castration-resistant prostate cancer (CRCP) is defined by disease progression despite androgen deprivation therapy (ADT). CRPC can be categorized as nonmetastatic or metastatic (mCRPC). mCRPC refers to CRPC that has spread beyond the prostate gland to a distant site, such as lymph nodes or bone. The progression of CRCP can encompass as any combination of a rise in serum prostate-specific antigen (PSA), progression of pre-existing disease, and appearance of initial or new metastases. Most CRPCs select mechanisms that upregulate intracellular androgens and/or androgen receptor (AR), leading to ongoing AR-directed cancer growth despite a castrate level of serum androgens. Thus, when patients develop CRPC they are usually sensitive to sequential "secondary" hormonal therapies (antiandrogens, ketoconazole, estrogens) directed at AR inhibition.

As used herein, the term "reference value" in the context of the number of CTCs expressing PSMA or the prevalence of a CTC subpopulation expressing PSMA, refers to the number or prevalence of the CTC subpopulation expressing PSMA in: (a) one or more corresponding samples obtained from the same patient at an earlier timepoint; (a) one or more corresponding samples obtained from the a similarly situated patient or average of patients at an earlier or at a corresponding timepoint; (c) one or more control/normal samples obtained from normal, or healthy, subjects, e.g. from males who do not have prostate cancer; or (d) any other corresponding reference standard deemed useful by one skilled in the art.

In some embodiments, determining the "number of CTCs expressing PSMA" refers to determining the absolute number. In other embodiments of the methods described herein the "number of CTCs expressing PSMA" can further encompass determining the relative number/proportion of CTCs expressing PSMA among all CTCs. In some embodiments, the absolute number or relative number/proportion of CTCs expressing PSMA among all CTCs is further referred to as the size of a subpopulation of CTCs expressing PSMA.

As used herein, the term "prevalence" in regards to a CTC subpopulation associated with CRPC in a test biological sample refers to the number of CTCs in the sample that belong to the CTC subpopulation.

As described herein, an increased prevalence of CTCs expressing PSMA is indicative of resistance to AR targeted therapy in a patient. As further described herein, an increased prevalence CTCs expressing PSMA is indicative response to PSMA targeted therapy in a patient. While both traditional and apoptotic CTCs are subpopulations of CTCs and encompassed in the term "PSMA expressing CTC" as defined herein, it is the increase in the relative proportion of either or both of these subpopulations among all CTCs in a sample that is indicative of resistance to AR targeted therapy in a patient. Similarly, an increase in the relative proportion of either or both of these subpopulations among all CTCs in a sample identifies a patient as a suitable candidate for PSMA targeted therapy. In its broadest sense, a biological sample can be any sample that contains CTCs

As used herein, the term "resistance" in the context of AR targeted therapy means that the subject does not show a response to the therapy based on an underlying ability of tumor cells to escape the effect of the therapeutic agent. Resistance includes de novo resistance and acquired resistance. Cancer patients that exhibit de novo resistance do not respond to a therapy from the start. However, in acquired resistance, the cancer cells initially respond to a drug but eventually acquire resistance to it. The cells might also show cross-resistance to other structurally and mechanistically unrelated drugs-a phenomenon commonly known as multi drug resistance (MDR). Owing to acquisition of MDR, treatment regimens that combine multiple agents with different targets are no longer effective

A sample can comprise a bodily fluid such as blood; the soluble fraction of a cell preparation, or an aliquot of media in which cells were grown; a chromosome, an organelle, or membrane isolated or extracted from a cell; genomic DNA, RNA, or cDNA in solution or bound to a substrate; a cell; a tissue; a tissue print; a fingerprint; cells; skin, and the like. A biological sample obtained from a subject can be any sample that contains nucleated cells and encompasses any material in which CTCs can be detected. A sample can be, for example, whole blood, plasma, saliva or other bodily fluid or tissue that contains cells.

In the method of the invention, the biological sample is a blood sample. As described herein, a sample can be whole blood, more preferably peripheral blood or a peripheral blood cell fraction. As will be appreciated by those skilled in the art, a blood sample can include any fraction or component of blood, without limitation, T-cells, monocytes, neutrophiles, erythrocytes, platelets and microvesicles such as exosomes and exosome-like vesicles. In the context of this disclosure, blood cells included in a blood sample encompass any nucleated cells and are not limited to components of whole blood. As such, blood cells include, for example, both white blood cells (WBCs) as well as rare cells, including CTCs.

The samples of this disclosure can each contain a plurality of cell populations and cell subpopulation that are distinguishable by methods well known in the art (e.g., FACS, immunohistochemistry). For example, a blood sample can contain populations of non-nucleated cells, such as erythrocytes (e.g., 4-5 million/µl) or platelets (150,000-400,000 cells/µl), and populations of nucleated cells such as WBCs (e.g., 4,500 - 10,000 cells/µl), CECs or CTCs (circulating tumor cells; e.g., 2-800 cells/). WBCs may contain cellular subpopulations of, e.g., neutrophils (2,500-8,000 cells/µl), lymphocytes (1,000-4,000 cells/µl), monocytes (100-700 cells/µl), eosinophils (50-500 cells/µl), basophils (25 - 100 cells/ ul) and the like. The samples of this disclosure are non-enriched samples, i.e., they are not enriched for any specific population or subpopulation of nucleated cells. For example, non-enriched blood samples are not enriched for CTCs, WBC, B-cells, T-cells, NK-cells, monocytes, or the like.

In some embodiments, the sample is a blood sample, obtained from a subject who has been diagnosed with prostate cancer based on tissue or liquid biopsy and/or surgery or clinical grounds. In some embodiments, the blood sample is obtained from a subject showing a clinical manifestation of prostate cancer advancing to CRPC, including without limitation, rising PSA levels prior to diagnosis, after initial surgery or radiation, or despite hormone therapy. In some embodiments, the sample is obtained from a subject who has been on hormone therapy or who has had a bilateral orchiectomy and whose testosterone levels have dropped to less than 50 ng/dl, and who shows evidence of disease progression in the form of rising PSA levels or bone or soft tissue metastases. In some cases, the sample is obtained from a subject who has been undergoing primary hormone therapies, which are the LHRH agonists, for example, leuprolide (Lupron) or goserelin (Zoladex). In some cases, the sample is obtained from a subject who has been undergoing AR targeted therapies, for example, Zytiga (arbiterone), which blocks androgen production, and Xtandi (enzalutamide), an androgen receptor (AR) inhibitor. In other embodiments, the biological sample is obtained from a healthy subject or a subject deemed to be at high risk for prostate cancer and/or metastasis of existing prostate cancer based on art known clinically established criteria including, for example, age, race, family and history.

As used herein, the term "direct analysis" means that the CTCs are detected in the context of all surrounding nucleated cells present in the sample as opposed to after enrichment of the sample for CTCs prior to detection. In some embodiments, the methods comprise microscopy providing a field of view that includes both CTCs and at least 200 surrounding white blood cells (WBCs).

In some embodiments, determining the number of CTCs expressing PSMA refers to determining the absolute number. In other embodiments of the methods described herein the number of CTCs expressing PSMA can further encompass determining the relative number/proportion of CTCs expressing PSMA among all CTCs. In some embodiments, the absolute number or relative number/proportion of CTCs expressing PSMA among all CTCs is further referred to as the size of a subpopulation of CTCs expressing PSMA.

A fundamental aspect of the present disclosure is the unparalleled robustness of the disclosed methods with regard to the detection of CTCs. The rare event detection disclosed herein with regard to CTCs is based on a direct analysis, *i.e.* non-enriched, of a population that encompasses the identification of rare events in the context of the surrounding non-rare events. Identification of the rare events according to the disclosed methods inherently identifies the surrounding events as non-rare events. Taking into account the surrounding non-rare events and determining the averages for non-rare events, for example, average cell size of non-rare events, allows for calibration of the detection method by removing noise. The result is a robustness of the disclosed methods that cannot be achieved with methods that are not based on direct analysis, but that instead compare enriched populations with inherently distorted contextual comparisons of rare events. The robustness of the direct analysis methods disclosed herein enables characterization of CTCs, including subpopulations of CTCs described herein, that cannot be achieved with other, enrichment-dependent CTC detection methods and that enables the identification and analysis of morphological and protein biomarkers indicative of the presence of a CTC subpopulation associated with CRPC in the context of the claimed methods. Approaches that enrich CTCs based on epithelial expression or physical characteristics are likely to miss non-traditional CTCs. Enumeration and characterization of non-traditional CTCs in mCRPC and other cancers provides prognostic/predictive information beyond traditional CTCs.

As described herein, the methods disclosed herein enable detection of CTCs expressing PSMA in a patient afflicted with prostate cancer, for example, mCRPC, and make it possible to distinguish between different patient groups in the resistance setting in order to tailor subsequent treatment more precisely and effectively. The methods of the invention further allow for resistance monitoring of a prostate cancer patients by enabling detection of an emergence of resistance to AR targeted therapies in a patient afflicted with prostate cancer. The rapid evolution of drug therapies in prostate cancer has vastly improved upon the use of docetaxel since its pivotal US Food and Drug Administration (FDA) approval in 2004 and has brought about a new era where progress has been made beyond the use of androgen deprivation therapy (ADT) with the addition of novel hormonal agents, immunotherapy, second-line chemotherapy as well as radiopharmaceuticals. The choice of sequencing currently relies on patient profiles, whether symptoms of metastatic disease exist or not. While survival outcomes are undeniably improved with the use of these therapies, disease will ultimately progress on each regimen.

Androgens in the form of testosterone or the more potent dihydrotestosterone (DHT) have been well-defined drivers of progression of prostate cancer and differentiation of the prostate gland. As such, the backbone of treatment for advanced prostate cancers was established decades ago when castration in the form of surgical orchiectomy achieved significant prostate tumor regression. Since then, substitution to chemical castration has been employed mostly due to patient preference. ADT has therefore become the standard systemic treatment for locally advanced or metastatic prostate cancer. While ADT is almost always effective in most patients, disease progression to castration resistance inevitably occurs. It is now increasingly recognized that the androgen receptor (AR) remains overexpressed despite seemingly castrate levels of testosterone, since alternative receptors may activate the AR or other target genes may help perpetuate the castrate-resistant phenotype, hence the term "castration-resistance" has become widely adopted in the literature. The enhanced understanding of the role of these androgens in stimulating the growth of prostate cancer has led to the development and approval of a newer generation AR targeted therapies such as Zytiga (arbiterone), which blocks androgen production, and Xtandi (enzalutamide), an androgen receptor (AR) inhibitor.

PSMA is a dimeric type II integral membrane glycoprotein, highly expressed on prostate cancer cells. It has been shown to be a predictor for prostate cancer progression and for prognosis of prostate cancer. For example, elevated PSMA levels are seen in aggressive forms of the disease, i.e., metastatic and higher-grade prostate cancers. High-level PSMA expression is correlated with early PSA recurrence in surgically treated prostate cancer. PSMA correlates with the aggressiveness of the disease, androgen receptor (AR) targeted therapy failure and progressive disease, and thereby strongly support PSMA as a target for prostate cancer characterization and subsequent therapy.

As described herein, the methods of the invention enable detection of CTCs expressing PSMA in a patient afflicted with mCRPC and make it possible to tailor subsequent treatment more precisely and effectively. The methods of the invention further allow for resistance monitoring of a prostate cancer patient by enabling detection of an emergence of AR targeted therapy failure in a patient afflicted with prostate cancer based on the detection of PSMA expressing CTCs. Furthermore, the methods of the invention allow for selecting a prostate cancer patient that is a candidate for PSMA targeted therapy, for example, BIND-014, Progenics ADC as well as other antibody based therapies targeting the exposed, extracellular domain of PSMA.

In some embodiments of the methods disclosed herein, the patient is undergoing hormone treatment. In certain embodiments, the hormone treatment is primary ADT. In additional embodiments, the patient is undergoing secondary hormone treatment and/or cytotoxic therapy. In some embodiments, the patient is undergoing a first "secondary" hormone therapy, such as antiandrogens and ketoconazole, which are options for nonmetastatic CRPC. In other embodiments, the patient is undergoing treatment with a second-generation RR targeted therapy such as Enzalutamide (Xtandi), which is more potent than first-generation antiandrogens because of its ability to block nuclear translocation of AR and approved for use in mCRPC, or abiraterone (Zytiga), which is a potent androgen synthesis inhibitor. In some embodiments, the patient is undergoing cytotoxic chemotherapy with a platinum-based regimen, for example and without limitation, docetaxel (Taxotere^{®}), mitoxantronepaclitaxel (Taxol^{®}) and cabazitaxel.

In some embodiments, the method for detecting PSMA on circulating tumor cells (CTCs) and related methods disclosed herein can further encompass individual patient risk factors, clinical, biopsy or imaging data, which includes any form of imaging modality known and used in the art, for example and without limitation, by X-ray computed tomography (CT), ultrasound, positron emission tomography (PET), electrical impedance tomography and magnetic resonance (MRI). It is understood that one skilled in the art can select an imaging modality based on a variety of art known criteria. Additionally, the methods disclosed herein, can optionally encompass one or more one or more individual risk factors that can be selected from the group consisting of, for example, age, race, family history, clinical history and/or data.

Risk factors for CRPC in the context of clinical data further include, for example, PSA, bone turnover markers, bone pain, bone scans. In those cases, biopsies can be performed to confirm or rule out mCRPC and methods for detecting mCRPC in a patient afflicted with prostate cancer can further encompass as a risk factor the resultant biopsy data. It is understood that one skilled in the art can select additional individual risk factors based on a variety of art known criteria. As described herein, the methods of the invention can encompass one or more individual risk factors. Accordingly, biomarkers can include, without limitation, imaging data, clinical data, biopsy data, and individual risk factors. As described herein, biomarkers also can include, but are not limited to, biological molecules comprising nucleotides, nucleic acids, nucleosides, amino acids, sugars, fatty acids, steroids, metabolites, peptides, polypeptides, proteins, carbohydrates, lipids, hormones, antibodies, regions of interest that serve as surrogates for biological macromolecules and combinations thereof (*e*.*g*., glycoproteins, ribonucleoproteins, lipoproteins) as well as portions or fragments of a biological molecule.

Direct analysis of CTCs as used in the invention includes both morphological features and immunofluorescent features. As will be understood by those skilled in the art, biomarkers can include a biological molecule, or a fragment of a biological molecule, the change and/or the detection of which can be correlated, individually or combined with other measurable features, with mCRPC. CTCs, which can be present as single cells or in clusters of CTCs, are often epithelial cells shed from solid tumors and are present in very low concentrations in the circulation of subjects. Accordingly, detection of CTCs in a blood sample can be referred to as rare event detection. CTCs have an abundance of less than 1:1,000 in a blood cell population, *e*.*g*., an abundance of less than 1:5,000, 1:10,000, 1:30,000, 1:50:000, 1:100,000, 1:300,000, 1:500,000, or 1:1,000,000. In some embodiments, the CTCs have an abundance of 1:50:000 to 1:100,000 in the cell population.

The samples of this disclosure may be obtained by any means, including, *e*.*g*., by means of solid tissue biopsy or fluid biopsy (*see*, *e.g.*, Marrinucci D. et al., 2012, Phys. Biol. 9(1):016003). Briefly, in particular embodiments, the process can encompass lysis and removal of the red blood cells in a 7.5 mL blood sample, deposition of the remaining nucleated cells on specialized microscope slides, each of which accommodates the equivalent of roughly 0.5 mL of whole blood. A blood sample may be extracted from any source known to include blood cells or components thereof, such as venous, arterial, peripheral, tissue, cord, and the like. The samples may be processed using well known and routine clinical methods (e.g., procedures for drawing and processing whole blood). In some embodiments, a blood sample is drawn into anti-coagulent blood collection tubes (BCT), which may contain EDTA or Streck Cell-Free DNA^{™}. In other embodiments, a blood sample is drawn into CellSave^{®} tubes (Veridex). A blood sample may further be stored for up to 12 hours, 24 hours, 36 hours, 48 hours, or 60 hours before further processing.

In some embodiments, the methods of this disclosure comprise an initial step of obtaining a white blood cell (WBC) count for the blood sample. In certain embodiments, the WBC count may be obtained by using a HemoCue^{®} WBC device (Hemocue, Ängelholm, Sweden). In some embodiments, the WBC count is used to determine the amount of blood required to plate a consistent loading volume of nucleated cells per slide and to calculate back the equivalent of CTCs per blood volume.

In some embodiments, the methods of this disclosure comprise an initial step of lysing erythrocytes in the blood sample. In some embodiments, the erythrocytes are lysed, *e.g.,* by adding an ammonium chloride solution to the blood sample. In certain embodiments, a blood sample is subjected to centrifugation following erythrocyte lysis and nucleated cells are resuspended, *e*.*g*., in a PBS solution.

In some embodiments, nucleated cells from a blood sample, are deposited as a monolayer on a planar support. The planar support may be of any material, *e.g.*, any fluorescently clear material, any material conducive to cell attachment, any material conducive to the easy removal of cell debris, any material having a thickness of < 100 µm. In some embodiments, the material is a film. In some embodiments' the material is a glass slide. In certain embodiments, the method encompasses an initial step of depositing nucleated cells from the blood sample as a monolayer on a glass slide. The glass slide can be coated to allow maximal retention of live cells (*See*, *e.g.*, Marrinucci D. et al., 2012, Phys. Biol. 9(1):016003). In some embodiments, about 0.5 million, 1 million, 1.5 million, 2 million, 2.5 million, 3 million, 3.5 million, 4 million, 4.5 million, or 5 million nucleated cells are deposited onto the glass slide. In some embodiments, the methods of this disclosure comprise depositing about 3 million cells onto a glass slide. In additional embodiments, the methods of this disclosure comprise depositing between about 2 million and about 3 million cells onto the glass slide. In some embodiments, the glass slide and immobilized cellular samples are available for further processing or experimentation after the methods of this disclosure have been completed.

In some embodiments, the methods of this disclosure comprise an initial step of identifying nucleated cells in the non-enriched blood sample. In some embodiments, the nucleated cells are identified with a fluorescent stain. In certain embodiments, the fluorescent stain comprises a nucleic acid specific stain. In certain embodiments, the fluorescent stain is diamidino-2-phenylindole (DAPI). In some embodiments, immunofluorescent staining of nucleated cells comprises pan cytokeratin (CK), cluster of differentiation (CD) 45 and DAPI. In some embodiments further described herein, CTCs comprise distinct immunofluorescent staining from surrounding nucleated cells. In some embodiments, the distinct immunofluorescent staining of CTCs detects DAPI (+), CK (+) and CD 45 (-) CTCs, also referred to as traditional CTCs. In some embodiments, the identification of CTCs further comprises comparing the intensity of pan cytokeratin fluorescent staining to surrounding nucleated cells. In some embodiments, the CTCs are CK- CTCs, that are identified as CTC based on other characteristics. As described herein, CTCs detected in the methods of the invention encompass traditional CTCs, cytokeratin negative (CK⁻) CTCs, small CTCs, and CTC clusters. In some embodiments, the CTC detection and analysis is accomplished by fluorescent scanning microscopy to detect immunofluorescent staining of nucleated cells in a blood sample. Marrinucci D. et al., 2012, Phys. Biol. 9(1):016003).

In particular embodiments, all nucleated cells are retained and immunofluorescently stained with monoclonal antibodies targeting cytokeratin (CK), an intermediate filament found exclusively in epithelial cells, a pan leukocyte specific antibody targeting the common leukocyte antigen CD45, and a nuclear stain, DAPI. The nucleated blood cells can be imaged in multiple fluorescent channels to produce high quality and high resolution digital images that retain fine cytologic details of nuclear contour and cytoplasmic distribution. While the surrounding WBCs can be identified with the pan leukocyte specific antibody targeting CD45, traditional CTCs can be identified, for example, as DAPI (+), CK (+) and CD 45 (-). In the methods described herein, the CTCs comprise distinct immunofluorescent staining from surrounding nucleated cells.

As described herein, CTCs encompass traditional CTCs, also referred to as high definition CTCs (HD-CTCs). Traditional CTCs are CK positive, CD45 negative, contain an intact DAPI positive nucleus without identifiable apoptotic changes or a disrupted appearance, and are morphologically distinct from surrounding white blood cells (WBCs). DAPI (+), CK (+) and CD45 (-) intensities can be categorized as measurable features during CTC enumeration as previously described. Nieva et al., Phys Biol 9:016004 (2012). The enrichment-free, direct analysis employed by the methods disclosed herein results in high sensitivity and high specificity, while adding high definition cytomorphology to enable detailed morphologic characterization of a CTC population known to be heterogeneous. In some embodiments, the morphological characteristics of a CTC detected in the methods of the invention comprise one or more of the group consisting of nucleus size, nucleus shape, presence of holes in nucleus, cell size, cell shape and nuclear to cytoplasmic ratio, nuclear detail, nuclear contour, prevalence of nucleoli, quality of cytoplasm and quantity of cytoplasm.

While traditional CTCs can be immunofluorescently identified as comprising DAPI (+), CK (+) and CD 45 (-) cells, the methods of the invention can be practiced with any other biomarkers that one of skill in the art selects for detecting traditional and non-traditional CTCs in a biological sample. One skilled in the art knows how to select a morphological feature, biological molecule, or a fragment of a biological molecule, the change and/or the detection of which can be correlated with a CTC. Molecule biomarkers include, but are not limited to, biological molecules comprising nucleotides, nucleic acids, nucleosides, amino acids, sugars, fatty acids, steroids, metabolites, peptides, polypeptides, proteins, carbohydrates, lipids, hormones, antibodies, regions of interest that serve as surrogates for biological macromolecules and combinations thereof (e.g., glycoproteins, ribonucleoproteins, lipoproteins). The term also encompasses portions or fragments of a biological molecule, for example, peptide fragment of a protein or polypeptide.

A person skilled in the art will appreciate that a number of methods can be used to detect and analyze CTCs, including microscopy based approaches including fluorescence scanning microscopy (*see*, *e.g.*, Marrinucci D. et al., 2012, Phys. Biol. 9(1):016003), mass spectrometry approaches, such as MS/MS, LC-MS/MS, multiple reaction monitoring (MRM) or SRM and product-ion monitoring (PIM) and also including antibody based methods such as immunofluorescence, immunohistochemistry, immunoassays such as Western blots, enzyme-linked immunosorbant assay (ELISA), immunopercipitation, radioimmunoassay, dot blotting, and FACS. Immunoassay techniques and protocols are generally known to those skilled in the art ( Price and Newman, Principles and Practice of Immunoassay, 2nd Edition, Grove's Dictionaries, 1997; and Gosling, Immunoassays: A Practical Approach, Oxford University Press, 2000.) A variety of immunoassay techniques, including competitive and non-competitive immunoassays, can be used (Self et al., Curr. Opin. Biotechnol., 7:60-65 (1996), *see also* John R. Crowther, The ELISA Guidebook, 1st ed., Humana Press 2000, ISBN 0896037282 and, An Introduction to Radioimmunoassay and Related Techniques, by Chard T, ed., Elsevier Science 1995, ISBN 0444821198).

A person of skill in the art will further appreciate that the prevalence of protein biomarkers may be detected using any class of marker-specific binding reagents known in the art, including, e.g., antibodies, aptamers, fusion proteins, such as fusion proteins including protein receptor or protein ligand components, or biomarker-specific small molecule binders. In some embodiments, the prevalence of AR, CK or CD45 is determined by an antibody.

The antibodies of this disclosure bind specifically to a protein biomarker. The antibody can be prepared using any suitable methods known in the art. *See, e.g.,* Coligan, Current Protocols in Immunology (1991); Harlow & Lane, Antibodies: A Laboratory Manual (1988); Goding, Monoclonal Antibodies: Principles and Practice (2d ed. 1986). The antibody can be any immunoglobulin or derivative thereof, whether natural or wholly or partially synthetically produced. All derivatives thereof which maintain specific binding ability are also included in the term. The antibody has a binding domain that is homologous or largely homologous to an immunoglobulin binding domain and can be derived from natural sources, or partly or wholly synthetically produced. The antibody can be a monoclonal or polyclonal antibody. In some embodiments, an antibody is a single chain antibody. Those of ordinary skill in the art will appreciate that antibody can be provided in any of a variety of forms including, for example, humanized, partially humanized, chimeric, chimeric humanized, *etc.* The antibody can be an antibody fragment including, but not limited to, Fab, Fab', F(ab')2, scFv, Fv, dsFv diabody, and Fd fragments. The antibody can be produced by any means. For example, the antibody can be enzymatically or chemically produced by fragmentation of an intact antibody and/or it can be recombinantly produced from a gene encoding the partial antibody sequence. The antibody can comprise a single chain antibody fragment. Alternatively or additionally, the antibody can comprise multiple chains which are linked together, for example, by disulfide linkages, and any functional fragments obtained from such molecules, wherein such fragments retain specific-binding properties of the parent antibody molecule. Because of their smaller size as functional components of the whole molecule, antibody fragments can offer advantages over intact antibodies for use in certain immunochemical techniques and experimental applications.

A detectable label can be used in the methods described herein for direct or indirect detection of the biomarkers when practicing the methods of the invention. A wide variety of detectable labels can be used, with the choice of label depending on the sensitivity required, ease of conjugation with the antibody, stability requirements, and available instrumentation and disposal provisions. Those skilled in the art are familiar with selection of a suitable detectable label based on the assay detection of the biomarkers in the methods of the invention. Suitable detectable labels include, but are not limited to, fluorescent dyes (*e.g.,* fluorescein, fluorescein isothiocyanate (FITC), Oregon Green^{™}, rhodamine, Texas red, tetrarhodimine isothiocynate (TRITC), Cy3, Cy5, Alexa Fluor^{®} 647, Alexa Fluor^{®} 555, Alexa Fluor^{®} 488), fluorescent markers (*e*.*g*.*,* green fluorescent protein (GFP), phycoerythrin, etc.), enzymes (*e*.*g*.*,* luciferase, horseradish peroxidase, alkaline phosphatase, etc.), nanoparticles, biotin, digoxigenin, metals, and the like.

For mass-sectrometry based analysis, differential tagging with isotopic reagents, *e*.*g*., isotope-coded affinity tags (ICAT) or the more recent variation that uses isobaric tagging reagents, iTRAQ (Applied Biosystems, Foster City, Calif.), followed by multidimensional liquid chromatography (LC) and tandem mass spectrometry (MS/MS) analysis can provide a further methodology in practicing the methods of this disclosure.

A chemiluminescence assay using a chemiluminescent antibody can be used for sensitive, non-radioactive detection of proteins. An antibody labeled with fluorochrome also can be suitable. Examples of fluorochromes include, without limitation, DAPI, fluorescein, Hoechst 33258, R-phycocyanin, B-phycoerythrin, R-phycoerythrin, rhodamine, Texas red, and lissamine. Indirect labels include various enzymes well known in the art, such as horseradish peroxidase (HRP), alkaline phosphatase (AP), beta-galactosidase, urease, and the like. Detection systems using suitable substrates for horseradish-peroxidase, alkaline phosphatase, beta.-galactosidase are well known in the art.

A signal from the direct or indirect label can be analyzed, for example, using a microscope, such as a fluorescence microscope or a fluorescence scanning microscope. Alternatively, a spectrophotometer can be used to detect color from a chromogenic substrate; a radiation counter to detect radiation such as a gamma counter for detection of ¹²⁵I, or a fluorometer to detect fluorescence in the presence of light of a certain wavelength. If desired, assays used to practice the methods of this disclosure can be automated or performed robotically, and the signal from multiple samples can be detected simultaneously.

In some embodiments, the biomarkers are immunofluorescent markers. In some embodiments, the immunofluorescent makers comprise a marker specific for epithelial cells In some embodiments, the immunofluorescent makers comprise a marker specific for white blood cells (WBCs). In some embodiments, one or more of the immunofluorescent markers comprise CD45 and CK.

In some embodiments, the prevalence of immunofluorescent markers in nucleated cells, such as CTCs or WBCs, results in distinct immunofluorescent staining patterns. Immunofluorescent staining patterns for CTCs and WBCs may differ based on which epithelial or WBC markers are detected in the respective cells. In some embodiments, determining prevalence of one or more immunofluorescent markers comprises comparing the distinct immunofluorescent staining of CTCs with the distinct immunofluorescent staining of WBCs using, for example, immunofluorescent staining of CD45, which distinctly identifies WBCs. There are other detectable markers or combinations of detectable markers that bind to the various subpopulations of WBCs. These may be used in various combinations, including in combination with or as an alternative to immunofluorescent staining of CD45.

CTCs may comprise distinct morphological characteristics compared to surrounding nucleated cells. In some instances, the morphological characteristics comprise nucleus size, nucleus shape, cell size, cell shape, and/or nuclear to cytoplasmic ratio. In some instances, the nucleated cells may be analyzed by nuclear detail, nuclear contour, prevalence of nucleoli, quality of cytoplasm, quantity of cytoplasm, intensity of immunofluorescent staining patterns. A person of ordinary skill in the art understands that the morphological characteristics of this disclosure may include any feature, property, characteristic, or aspect of a cell that can be determined and correlated with the detection of a CTC. The morphological characteristics may comprise one or more of the group consisting of nucleus size, nucleus shape, presence of holes in nucleus, cell size, cell shape and nuclear to cytoplasmic ratio, nuclear detail, nuclear contour, prevalence of nucleoli, quality of cytoplasm and quantity of cytoplasm.

Detection and analysis of CTCs can be performed with any suitable microscopic method known in the art. In some embodiments, the method is performed by fluorescent scanning microscopy. In certain embodiments, the microscopic method provides high-resolution images of CTCs and their surrounding WBCs (*see*, *e.g.*, Marrinucci D. et al., 2012, Phys. Biol. 9(1):016003). In some embodiments, a slide coated with a monolayer of nucleated cells from a sample, such as a non-enriched blood sample, is scanned by a fluorescent scanning microscope and the fluorescence intensities from immunofluorescent markers and nuclear stains are recorded to allow for the determination of the prevalence of each immunofluorescent marker and the assessment of the morphology of the nucleated cells. In some embodiments, microscopic data collection and analysis is conducted in an automated manner.

In some embodiments, the methods of the invention include detecting one or more biomarkers, for example, AR, CK and CD 45. A biomarker is considered present in a cell if it is detectable above the background noise of the respective detection method used (*e.g.*, 2-fold, 3-fold, 5-fold, or 10-fold higher than the background; *e*.*g*., 2σ or 3σ over background). In some embodiments, a biomarker is considered absent if it is not detectable above the background noise of the detection method used (*e.g.*, <1.5-fold or <2.0-fold higher than the background signal; *e.g.*, <1.5σ or <2.0σ over background).

In some embodiments, the prevalence of immunofluorescent markers in nucleated cells is determined by selecting the exposure times during the fluorescence scanning process such that all immunofluorescent markers achieve a pre-set level of fluorescence on the WBCs in the field of view. Under these conditions, CTC-specific immunofluorescent markers, even though absent on WBCs are visible in the WBCs as background signals with fixed heights. Moreover, WBC-specific immunofluorescent markers that are absent on CTCs are visible in the CTCs as background signals with fixed heights. A cell is considered positive for an immunofluorescent marker (*i.e.*, the marker is considered present) if its fluorescent signal for the respective marker is significantly higher than the fixed background signal (*e.g.*, 2-fold, 3-fold, 5-fold, or 10-fold higher than the background; *e*.*g*., 2σ or 3σ over background). For example, a nucleated cell is considered CD 45 positive (CD 45⁺) if its fluorescent signal for CD 45 is significantly higher than the background signal. A cell is considered negative for an immunofluorescent marker (*i.e.*, the marker is considered absent) if the cell's fluorescence signal for the respective marker is not significantly above the background signal (*e.g.*, <1.5-fold or <2.0-fold higher than the background signal; *e.g.*, <1.5σ or <2.0σ over background).

Typically, each microscopic field contains both CTCs and WBCs. In certain cases, the microscopic field shows at least 1, 5, 10, 20, 50, or 100 CTCs. In certain cases , the microscopic field shows at least 10, 25, 50, 100, 250, 500, or 1,000 fold more WBCs than CTCs. In certain cases, the microscopic field comprises one or more CTCs or CTC clusters surrounded by at least 10, 50, 100, 150, 200, 250, 500, 1,000 or more WBCs.

In some methods described herein, detection of CTCs comprises enumeration of CTCs that are present in the blood sample. Some embodiments, the methods described herein encompass detection of at least 1.0 CTC/mL of blood, 1.5 CTCs/mL of blood, 2.0 CTCs/mL of blood, 2.5 CTCs/mL of blood, 3.0 CTCs/mL of blood, 3.5 CTCs/mL of blood, 4.0 CTCs/mL of blood, 4.5 CTCs/mL of blood, 5.0 CTCs/mL of blood, 5.5 CTCs/mL of blood, 6.0 CTCs/mL of blood, 6.5 CTCs/mL of blood, 7.0 CTCs/mL of blood, 7.5 CTCs/mL of blood, 8.0 CTCs/mL of blood, 8.5 CTCs/mL of blood, 9.0 CTCs/mL of blood, 9.5 CTCs/mL of blood, 10 CTCs/mL of blood, or more.

In some embodiments, the CTCs detected in a biological sample comprise distinct subtypes of CTCs, including non-traditional CTCs. In some embodiments, the methods described herein encompass detection of at least 0.1 CTC cluster/mL of blood, 0.2 CTC clusters/mL of blood, 0.3 CTC clusters/mL of blood, 0.4 CTC clusters/mL of blood, 0.5 CTC clusters/mL of blood, 0.6 CTC clusters/mL of blood, 0.7 CTC clusters/mL of blood, 0.8 CTC clusters/mL of blood, 0.9 CTC clusters/mL of blood, 1 CTC cluster/mL of blood, 2 CTC clusters/mL of blood, 3 CTC clusters/mL of blood, 4 CTC clusters/mL of blood, 5 CTC clusters/mL of blood, 6 CTC clusters/mL of blood, 7 CTC clusters/mL of blood, 8 CTC clusters/mL of blood, 9 CTC clusters/mL of blood, 10 clusters/mL or more. In a particular embodiment, the methods described herein encompass detection of at least 1 CTC cluster/mL of blood

The method for detecting PSMA on circulating tumor cells (CTCs) and related methods disclosed herein may further comprise molecular characterization of the CTCs, for example, by fluorescence *in situ* hybridization (FISH). Standard molecular biology techniques known in the art and not specifically described are generally followed as in Sambrook et al., Molecular Cloning: A Laboratory Manual, Cold Spring Harbor Laboratory Press, New York (1989), and as in Ausubel et al., Current Protocols in Molecular Biology, John Wiley and Sons, Baltimore, Md. (1989) and as in Perbal, A Practical Guide to Molecular Cloning, John Wiley & Sons, New York (1988), and as in Watson et al., Recombinant DNA, Scientific American Books, New York and in Birren et al (eds) Genome Analysis: A Laboratory Manual Series, Vols. 1-4 Cold Spring Harbor Laboratory Press, New York (1998). Polymerase chain reaction (PCR) can be carried out generally as in PCR Protocols: A Guide to Methods and Applications, Academic Press, San Diego, Calif. (1990). Any method capable of determining a DNA copy number profile of a particular sample can be used for molecular profiling provided the resolution is sufficient to identify the biomarkers . The skilled artisan is aware of and capable of using a number of different platforms for assessing whole genome copy number changes at a resolution sufficient to identify the copy number of the one or more biomarkers

*In situ* hybridization assays are well known and are generally described in Angerer et al., Methods Enzymol. 152:649-660 (1987). In an in situ hybridization assay, cells, e.g., from a biopsy, are fixed to a solid support, typically a glass slide. If DNA is to be probed, the cells are denatured with heat or alkali. The cells are then contacted with a hybridization solution at a moderate temperature to permit annealing of specific probes that are labeled. The probes are preferably labeled with radioisotopes or fluorescent reporters. FISH (fluorescence in situ hybridization) uses fluorescent probes that bind to only those parts of a sequence with which they show a high degree of sequence similarity.

FISH is a cytogenetic technique used to detect and localize specific polynucleotide sequences in cells. For example, FISH can be used to detect DNA sequences on chromosomes. FISH can also be used to detect and localize specific RNAs, e.g., mRNAs, within tissue samples. In FISH uses fluorescent probes that bind to specific nucleotide sequences to which they show a high degree of sequence similarity. Fluorescence microscopy can be used to find out whether and where the fluorescent probes are bound. In addition to detecting specific nucleotide sequences, e.g., translocations, fusion, breaks, duplications and other chromosomal abnormalities, FISH can help define the spatial-temporal patterns of specific gene copy number and/or gene expression within cells and tissues.

In some embodiments, the method for detecting PSMA on circulating tumor cells (CTCs) and related methods disclosed herein encompass the use of a predictive model. In further embodiments, the disclosed method for detecting PSMA on circulating tumor cells (CTCs) obtained from a patient afflicted with prostate cancer as well as the related methods disclosed herein encompass comparing a measurable feature with a reference feature. As those skilled in the art can appreciate, such comparison can be a direct comparison to the reference feature or an indirect comparison where the reference feature has been incorporated into the predictive model. In further embodiments, analyzing a measurable feature in a method for detecting PSMA on circulating tumor cells (CTCs) obtained from a patient afflicted with prostate cancer as well as the related methods disclosed herein encompasses one or more of a linear discriminant analysis model, a support vector machine classification algorithm, a recursive feature elimination model, a prediction analysis of microarray model, a logistic regression model, a CART algorithm, a flex tree algorithm, a LART algorithm, a random forest algorithm, a MART algorithm, a machine learning algorithm, a penalized regression method, or a combination thereof. In particular embodiments, the analysis comprises logistic regression. In additional embodiments, the detection of mCRPC in a patient afflicted with prostate cancer is expressed as a risk score.

An analytic classification process can use any one of a variety of statistical analytic methods to manipulate the quantitative data and provide for classification of the sample. Examples of useful methods include linear discriminant analysis, recursive feature elimination, a prediction analysis of microarray, a logistic regression, a CART algorithm, a FlexTree algorithm, a LART algorithm, a random forest algorithm, a MART algorithm, machine learning algorithms and other methods known to those skilled in the art.

Classification can be made according to predictive modeling methods that set a threshold for determining the probability that a sample belongs to a given class. The probability preferably is at least 50%, or at least 60%, or at least 70%, or at least 80%, or at least 90% or higher. Classifications also can be made by determining whether a comparison between an obtained dataset and a reference dataset yields a statistically significant difference. If so, then the sample from which the dataset was obtained is classified as not belonging to the reference dataset class. Conversely, if such a comparison is not statistically significantly different from the reference dataset, then the sample from which the dataset was obtained is classified as belonging to the reference dataset class.

The predictive ability of a model can be evaluated according to its ability to provide a quality metric, e.g. AUROC (area under the ROC curve) or accuracy, of a particular value, or range of values. Area under the curve measures are useful for comparing the accuracy of a classifier across the complete data range. Classifiers with a greater AUC have a greater capacity to classify unknowns correctly between two groups of interest. ROC analysis can be used to select the optimal threshold under a variety of clinical circumstances, balancing the inherent tradeoffs that exist between specificity and sensitivity. In some embodiments, a desired quality threshold is a predictive model that will classify a sample with an accuracy of at least about 0.7, at least about 0.75, at least about 0.8, at least about 0.85, at least about 0.9, at least about 0.95, or higher. As an alternative measure, a desired quality threshold can refer to a predictive model that will classify a sample with an AUC of at least about 0.7, at least about 0.75, at least about 0.8, at least about 0.85, at least about 0.9, or higher.

As is known in the art, the relative sensitivity and specificity of a predictive model can be adjusted to favor either the specificity metric or the sensitivity metric, where the two metrics have an inverse relationship. The limits in a model as described above can be adjusted to provide a selected sensitivity or specificity level, depending on the particular requirements of the test being performed. One or both of sensitivity and specificity can be at least about 0.7, at least about 0.75, at least about 0.8, at least about 0.85, at least about 0.9, or higher.

The raw data can be initially analyzed by measuring the values for each measurable feature or biomarker, usually in triplicate or in multiple triplicates. The data can be manipulated, for example, raw data can be transformed using standard curves, and the average of triplicate measurements used to calculate the average and standard deviation for each patient. These values can be transformed before being used in the models, *e.g.* log-transformed, Box-Cox transformed (Box and Cox, Royal Stat. Soc., Series B, 26:211-246(1964). The data are then input into a predictive model, which will classify the sample according to the state. The resulting information can be communicated to a patient or health care provider.

In some embodiments, the method for detecting PSMA on circulating tumor cells (CTCs) and related methods disclosed herein have a specificity of >60%, >70%, >80%, >90% or higher. In additional embodiments, the method for detecting PSMA on circulating tumor cells (CTCs) and related methods disclosed herein have a specificity >90% at a classification threshold of 7.5 CTCs/mL of blood.

The following examples are provided by way of illustration, not limitation.

### EXAMPLES

### Example 1. Detection of PSMA on CTCs surface comprising HD Imaging

This experiment demonstrates detection of PSMA on CTCs surface comprising high definition imaging of plated nucleated cells based on a direct analysis comprising immunofluorescent staining and morphological characteristics of the nucleated cells.

Blood samples were obtained from mCRPC patients (pts). Cells were stained for CK, CD45, PSMA and categorized as traditional CTC (CK+, CD45-, intact/morphologically distinct nuclei) or apoptotic CTC (CK+, CD45-, morphology suggesting apoptosis). Clinical data including treatment, metastatic sites, Veridex CTC count, PSA, and alkaline phosphatase was collected.

Fourteen pts with mCRPC, including 8 with serial samples were analyzed (33 samples in total). At the first draw (t1), traditional CTC were detected in 13 pts (93%), (median 2.5cells/ml, range 0-43) and apoptotic CTC in 14 pts (100%) (median 4.5 cells/ml, range 1-32) including 6 pts (42%) with no CTC by Veridex CellSearch^{®} PSMA expression was detected in 5 pts (36%) with traditional CTC of which a median of 33% of cells (range 32-100%) expressed the antigen. Similar intra-patient heterogeneity was seen for the 10 pts (71.4%) with PSMA+ apoptotic CTCs (median 33%, range 11-75% cells).

During treatment, often with more complete androgen suppression (at t1: enzalutamide (n=2), abiraterone (n=4), ADT alone (n=1), Casodex (n=1), PSMA was later detected in 3 of the 8 (38%) pts with no PSMA+ traditional CTCs at t1. The presence of PSMA expression in apoptotic CTCs did not change.

PSMA was later detected in 3 of the 8 (38%) pts with no PSMA+ traditional CTCs at t1; these 3 pts were all receiving abiraterone. The presence of PSMA expression in apoptotic CTCs did not change.

A larger percentage of PSMA expression was seen in mCRPC patients with apoptotic CTC (10/14) than traditional CTC (5/14) at t1. Intra-patient cell heterogeneity of PSMA expression existed in both traditional and apoptotic CTCs. Serial measures suggest dynamic changes in PSMA expression over time. Larger samples of patients at discrete time points on therapy are underway to further elucidate the potential clinical relevance.

### Example 2. Heterogeneity of PSMA expression in traditional and apoptotic CTCs in mCRPC

36 patients (pts) with metastatic castration resistant prostate cancer (mCRPC) had blood drawn as part of an IRB approved biospecimen protocol, with a total of 92 samples collected and shipped to Epic Sciences. The first timepoint was designated as t1. Circulating tumor cells were identified utilizing the Epic CTC collection and detection process (Figure 1). Traditional or classic CTCs were identified as CK+CD45- cells with intact DAPI nuclei after pathologist review of their morphology while apoptotic CTCs were defined as those CK+CD45- cells with morphology consistent with apoptosis. After enumeration of all cells, candidate CTCs from both traditional and apoptotic cells were then evaluated for the expression of PSMA using immunofluorescence (IF). Clinical characteristics, including current and prior therapy history, PSA, and concomitant enumeration byCellSearch, were collected for each sample.

6/14 (42.9%) of pts with CTCs that did not express PSMA at t1, ultimately did have PSMA expression in CTCs on a subsequent timepoint. Almost all (5/6) cases of "conversion" from PSMA - to + CTCs were associated with a change in therapy prompted by disease progression. 8/14 (57.1%) of pts with CTCs that did not express PSMA at t1 continued to have PSMA negative CTC on serial draws. Half of these pts remained on the same therapy, and half changed treatment. In contrast, 10/13 (76.9%) pts with CTCs expressing PSMA at t1, continued to have PSMA expressing CTCs at subsequent timepoints. The 3 pts (3/13) with PSMA expressing CTCs at t1 who "converted" to CTCs that no longer expressed PSMA all did so with a change in therapy (Figure 4 highlights one case).

Exposure to prior chemotherapy did not appear to change the presence or absence of PSMA expressing CTCs: 12/27 (44.4%) chemotherapy naive patients had traditional CTCs that expressed PSMA at t1 in comparison to 5/9 (55.6%) patients with a history of prior chemotherapy exposure. Chemotherapy status also did not appear to alter the presence of PSMA + apoptotic CTCs (21/27 (77.8%) vs 6/9 (66.7%) in those chemotherapy naive versus exposed patients, respectively.

PSMA expression was detected in both apoptotic CTCs as well as traditional CTCs. 81.5% and 40.2% of all samples drawn had CTCs that expressed PSMA in these two CTC populations, respectively. Intra-patient cell heterogeneity of PSMA expression was characteristic of both CTC populations. There are dynamic changes in PSMA expression over time, not only in patients changing therapy, but in patients continuing the same treatment. Conversion from PSMA+ CTCs to PSMA - CTCs was uncommon with serial blood draws, but when it did occur, this appeared to be related to change in therapy. The threshold of detectable cells, and the proportion and degree of PSMA expression that associates with drug sensitivity is unknown.

## Claims

1. A method for detecting prostate specific membrane antigen (PSMA) on circulating tumor cells (CTCs) obtained from a patient afflicted with prostate cancer comprising:
(a) performing a direct analysis comprising immunofluorescent staining and morphological characterization of nucleated cells in a blood sample obtained from the patient to detect circulating tumor cells (CTC), wherein the direct analysis means the detection of CTCs in the context of all surrounding nucleated cells present in the sample as opposed to after enrichment of the sample for CTCs prior to detection; and
(b) determining the number of CTCs expressing PSMA,
wherein the direct analysis in step (a) identifies more than one CTC subpopulation expressing PSMA,
wherein a first CTC subpopulation expressing PSMA comprises traditional CTCs,
wherein the traditional CTCs contain a diamidino-2-phenylindole (DAPI+) nucleus, are cytokeratin positive (CK+), and cluster of differentiation 45 negative (CD45-) and are morphologically distinct from surrounding white blood cells (WBCs), and
wherein a second CTC subpopulation expressing PSMA comprises CK+CD45- apoptotic CTCs.

2. The method of claim 1, wherein said apoptotic CTCs lack intact nuclei.

3. The method of claim 1, wherein the immunofluorescent staining of nucleated cells comprises pan cytokeratin, CD45, and DAPI.

4. The method of claim 1, wherein determining the presence of the CTC subpopulations expressing PSMA in step (b) comprises analysis of the CTCs detected in step (a) at the single cell level.

5. The method of claim 1, wherein the prostate cancer is metastatic castration-resistant prostate cancer (mCRPC).

6. The method of claim 1, wherein the direct analysis comprises fluorescent scanning microscopy.

## Patentansprüche

1. Verfahren zum Nachweis von prostataspezifischem Membranantigen (PSMA) auf zirkulierenden Tumorzellen (CTCs), die von einem an Prostatakrebs erkrankten Patienten gewonnen wurden, umfassend:
(a) Durchführen einer direkten Analyse, die Immunfluoreszenzfärbung und morphologische Charakterisierung von kernhaltigen Zellen in einer vom Patienten gewonnenen Blutprobe umfasst, um zirkulierende Tumorzellen (CTC) nachzuweisen, wobei die direkte Analyse den Nachweis von CTCs im Kontext aller umgebenden kernhaltigen Zellen bedeutet, die in der Probe vorhanden sind, im Gegensatz zur Anreicherung der Probe auf CTCs vor dem Nachweis; und
(b) Bestimmen der Anzahl von PSMA-exprimierenden CTCs,
wobei die direkte Analyse in Schritt (a) mehr als eine PSMA-exprimierende CTC-Subpopulation identifiziert,
wobei eine erste PSMA-exprimierende CTC-Subpopulation traditionelle CTCs umfasst,
wobei die traditionellen CTCs einen Diamidin-2-Phenylindol- (DAPI+) Kern enthalten, Cytokeratin-positiv (CK+) und Cluster of Differentiation 45-negativ (CD45-) sind und sich morphologisch von umgebenden weißen Blutkörperchen (WBCs) unterscheiden, und
wobei eine zweite PSMA-exprimierende CTC-Subpopulation CK+CD45-apoptotische CTCs umfasst.

2. Verfahren nach Anspruch 1, wobei die apoptotischen CTCs keine intakten Kerne besitzen.

3. Verfahren nach Anspruch 1, wobei die Immunfluoreszenzfärbung kernhaltiger Zellen Pan-Cytokeratin, CD45 und DAPI umfasst.

4. Verfahren nach Anspruch 1, wobei das Bestimmen des Vorhandenseins der PSMA-exprimierenden CTC-Subpopulationen in Schritt (b) die Analyse der in Schritt (a) nachgewiesenen CTCs auf Einzelzellebene umfasst.

5. Verfahren nach Anspruch 1, wobei es sich beim Prostatakrebs um metastasiertes kastrationsresistentes Prostatakarzinom (mCRPC) handelt.

6. Verfahren nach Anspruch 1, wobei die direkte Analyse Fluoreszenz-Rastermikroskopie umfasst.

## Revendications

1. Procédé de détection de l'antigène membranaire spécifique de la prostate (PSMA) sur des cellules tumorales circulantes (CTC) prélevées chez un patient atteint d'un cancer de la prostate comprenant :
(a) la réalisation d'une analyse directe comprenant la coloration immunofluorescente et la caractérisation morphologique de cellules nuclées dans un échantillon de sang prélevé chez le patient en vue de détecter des cellules tumorales circulantes (CTC), dans lequel l'analyse directe signifie la détection de CTC dans le contexte de toutes les cellules nuclées environnantes présentes dans l'échantillon par opposition à un post-enrichissement de l'échantillon pour CTC avant la détection ; et
(b) la détermination du nombre de CTC exprimant le PSMA,
dans lequel l'analyse directe de l'étape (a) identifie plus d'une sous-population de CTC exprimant le PSMA,
dans lequel une première sous-population de CTC exprimant le PSMA comprend des CTC classiques,
dans lequel les CTC classiques contiennent un noyau de diamidino-2-phénylindole (DAPI+), sont positives à la cytokératine (CK+) et négatives au cluster de différenciation 45 (CD45-) et sont morphologiquement distinctes de globules blancs (WBC) environnants, et
dans lequel une deuxième sous-population de CTC exprimant le PSMA comprend des CTC apoptotiques CK+CD45-.

2. Procédé selon la revendication 1, dans lequel lesdites CTC apoptotiques ne comportent pas de noyaux intacts.

3. Procédé selon la revendication 1, dans lequel la coloration par immunofluorescence de cellules nuclées comprend pan cytokératine, CD45 et DAPI.

4. Procédé selon la revendication 1, dans lequel la détermination de la présence des sous-populations de CTC exprimant le PSMA à l'étape (b) comprend l'analyse des CTC détectées à l'étape (a) au niveau de la cellule individuelle.

5. Procédé selon la revendication 1, dans lequel le cancer de la prostate est un cancer de la prostate résistant à la castration métastatique (CPRCm).

6. Procédé selon la revendication 1, dans lequel l'analyse directe comprend la microscopie à balayage fluorescent.
